(19) Europäisches Patentamt
European Patent Office
Office européen des brevets

(11) **EP 4 529 919 A1**

(12) **EUROPEAN PATENT APPLICATION**

(43) Date of publication:
**02.04.2025 Bulletin 2025/14**

(21) Application number: **23200805.2**

(22) Date of filing: **29.09.2023**

(51) International Patent Classification (IPC):
*A61K 9/107* (2006.01)     *A61K 47/38* (2006.01)
*B01D 39/18* (2006.01)     *C08L 1/02* (2006.01)
*C12N 15/09* (2006.01)     *C12P 19/04* (2006.01)

(52) Cooperative Patent Classification (CPC):
**A61K 9/1075; A61K 47/38; B01D 39/18;
C08L 1/02; C12P 19/04**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Evonik Operations GmbH
45128 Essen (DE)**

(72) Inventors:
• **SCHMIED, Fabian-Pascal
63225 Langen (DE)**

• **BERNHARDT, Alexander
55268 Nieder-Olm (DE)**
• **NOLLENBERGER, Kathrin
62495 Darmstadt (DE)**
• **ENDRES, Thomas
55131 Mainz (DE)**
• **BEEKMANN, Uwe
07745 Jena (DE)**

(74) Representative: **Evonik Patent Association
c/o Evonik Industries AG
IP Management
Postcode 84/339
Rodenbacher Chaussee 4
63457 Hanau (DE)**

(54) **SOLID SELF-NANOEMULSIFYING DRUG DELIVERY SYSTEM HAVING BIOSYNTHETIC CELLULOSE AS ADSORBENT**

(57) The presently claimed invention is directed to a solid self-nanoemulsifying drug delivery system, which comprises:
a. biosynthetic cellulose,
b. at least one pharmaceutically active ingredient, or at least one nutraceutical ingredient,
c. at least one lipid, and
d. at least one surfactant,
wherein the at least one pharmaceutically active ingredient, or the at least one nutraceutical ingredient is at least partially adsorbed on the biosynthetic cellulose.

EP 4 529 919 A1

**Description**

**Field of the invention**

The present invention is directed to a solid self-nanoemulsifying drug delivery system, which comprises biosynthetic cellulose.

**Background**

Self-nanoemulsifying drug delivery systems (SNEDDS) are well known in the field of pharmaceutical compositions. The systems use the emulsion of poorly water-soluble active ingredients to improve drug solubilization. In general, two kinds of SNEDDS are known, liquid or semi-liquid SNEDDS and solid SNEDDS (S-SNEDDS). While the liquid or semi-liquid character of SNEDDS is often seen as a disadvantage in view of dosing for peroral applications and in view of their lack of storage stability, solid self-nanoemulsifying systems are preferred.

General methods of providing solid self-nanoemulsifying drug delivery systems or in the neighboring field of self-micro emulsifying drug delivery systems (SMEDDS) are for example disclosed in:
EP 2 101 729 B1, which describes in this regard several ways for the conversion of microemulsions into the solid state. These are adsorption on colloidal silicon dioxide, stabilization of individual phases with colloidal silicon dioxide and hydrophobic colloidal silicon dioxide, incorporation in polyethylene glycol dispersions and spray drying.

Silva, D. Luis Antonio, et al. (International Journal of Pharmaceutics 541 (2018) 1 - 10), which describes a preparation of a solid self-micro emulsifying drug delivery system (S-SMEDDS) by hot melt extrusion. S-SMEDDS were prepared by blending carvedilol and a lipid mixture with hydroxyl propyl methyl cellulose acetate succinate (HPMCAS). Extrudates prepared at the lowest drug concentration and highest temperature and recirculation time promoted a complete and rapid drug release at pH 6.8.

CN107308133 A, which describes S-SMEDDS comprising curcumin containing SMEDDS employing AEROSIL® 200 as adsorbent.

The technical solutions disclosed in the prior art suffer from drawbacks such as lower loading of the pharmaceutically active ingredient, or the nutraceutical ingredient, and/or lower release of the pharmaceutically active ingredient, or the nutraceutical ingredient.

Thus, it is an object of the present invention to provide a S-SNEDDS with an improved/increased loading of the pharmaceutically active ingredient, or the nutraceutical ingredient. Another object of the present invention is to provide a S-SNEDDS with an improved/increased release of the pharmaceutically active ingredient or the nutraceutical ingredient. Yet another object of the present invention is to provide a S-SNEDDS with an improved/increased loading and release of the pharmaceutically active ingredient or the nutraceutical ingredient.

**Summary of the invention**

It was found that using a biosynthetic cellulose as adsorbent for the L-SNEDDS (Liquid self-nanoemulsifying drug delivery system) can not only improve the leading capacity of the S-SNEDDS but also improve the release rate of the pharmaceutically active ingredient or the nutraceutical ingredient.

Thus, in a first aspect, the present invention is directed to a composition comprising:

a. a biosynthetic cellulose,

b. a pharmaceutically active ingredient, or a nutraceutical ingredient,

c. at least one lipid, and

d. at least one surfactant,

wherein the pharmaceutically active ingredient, or the nutraceutical ingredient is at least partially adsorbed on the biosynthetic cellulose.

In a second aspect, the present invention is directed to a process for preparing a composition according to first aspect comprising the steps of:

a. providing at least one pharmaceutically active ingredient, or at least one nutraceutical ingredient,

b. providing at least one lipid,

c. providing at least one surfactant,

d. mixing the components of step a., step b., and step c. to obtain a homogenous mixture, and

e. providing biosynthetic cellulose to the homogenous mixture obtained in step d. to obtain the product.

In a third aspect, the present invention is directed to a method of delivering a pharmaceutically active ingredient, or a nutraceutical ingredient comprising at least the step of:

a. providing a composition according to first aspect.

## Detailed description of the invention

The term 'partially adsorbed' with the present invention means that at least 20%, or 40%, or 60%, or 80%, or preferably 90%, or 100% of the L-SNEDD is adsorbed on to the biosynthetic nano cellulose.
In an embodiment the present invention is directed to a composition comprising:

a. biosynthetic cellulose,
b. at least one pharmaceutically active ingredient, or at least one nutraceutical ingredient,
c. at least one lipid, and
d. at least one surfactant,

wherein the at least one pharmaceutically active ingredient, or the at least one nutraceutical ingredient is at least partially adsorbed on the biosynthetic cellulose. It is advantageous to use cellulose obtained from bacterial strain as it provides higher loading capacity for the L-SNEDDS and improve the release profile of the biologically active ingredient.

## Biosynthetic cellulose (a)

The biosynthetic cellulose according to present invention is obtained from a microorganism. The microorganism is preferably a bacterial microorganism and is selected from *Rhizobium, Pseudomonas, Azotobacter, Komagataeibacter xylinus (acetobacter), Agrobacteriu, Achromobacter, Sarcina,* or a genetically modified strain of any of the above listed microorganism.
The bacterial nanocellulose (BNC), which refers to a nano-structured cellulose produced by a bacterium or a genetically modified strain of it. The bacterial strain of Komagataeibacter xylinus or its genetically modified strain are known to be one of the best bacterial species which give the highest efficiency in cellulose production. The BNC is a biomaterial having unique properties such as: chemical purity, excellent mechanical strength, biodegradability, biocompatibility, high water-holding capability, high flexibility, high absorbency, possibility of forming any shape and size due to extraordinary formability, softness, and a high degree of polymerization. The BNC is considered non-cytotoxic, non-genotoxic, and highly biocompatible material. The BNC consists of randomly assembled, < 100 nm wide ribbon-shaped fibrils, composed of 7-8 nm wide elementary nanofibrils aggregated in bundles. This unique nano-morphology results in a large surface area.
The BNC exists as a network polymer, or a fiber polymer and has high purity. The problem associated with the cellulose obtained from plant is always contaminated with lignin, whereas the BNC obtained from microorganism is free of lignin. The BNC has density in the range of 0.5 to 1.3 $g/cm^3$ and has high degree of crystallinity ranging from 80 to 100 %, whereas the plant-based cellulose usually has crystallinity in the range of 40 to 60%. The crystallinity of the cellulose is determined according to solid state NMR spectroscopy. The cellulose of bacterial origin has surface area > 1 $m^2/g$ , preferably in the range of 1 to 200 $m^2/g$, and most preferably in the range of 15 to 100 $m^2/g$ as measured according to DIN ISO 9277.The weight average molecular weight (Mw) in the range of 100,000 to 2,000,000 g/mol as measured according to GPC. The preferred weight average molecular weight (Mw) of the BNC in the range of 500,000 to 780,000 g/mol as determined according to GPC. The BNC as fiber polymer has a diameter in the range of 1 nm to 5 nm and has a length in the range of 1 nm to 1000 nm.

## Method of producing Biosynthetic cellulose (a)

The different methods for producing BNC are known, however the widely used methods are the static cultivation process, the semi-static cultivation process, and the agitated cultivation process. The major method of production of the BNC today is by fermentation of coconut water in the static cultivation process.
A preferred method for the synthesis of nano cellulose from bacteria is described in EP 2547372 A1. It is preferred that at least two different cellulose-producing bacterial strains used together in a common culture medium. Thus, it is possible to modulate the BNC structure and the properties by the choice of the at least two different bacterial strains, by their preparation, and inoculation as well as by the synthetic conditions. It is also possible that the at least two different bacterial cellulose networks are prepared independently from each other and are subsequently brought together and are synthesized together. The preferred bacterial strain used for the fermentation are selected from the strains Acetobacter xylinus, K. xylinum, Gluconacetobacter hansenii NQ5 (Acetobacter xylinus Yamada) ATTC 53582, the strains of K. xylinum ATCC 11142, DSM 14666 or a mixture thereof. In preferred embodiments, the bacterial culture may comprise a mixture of two bacterial strains or even three bacterial strains. In a preferred embodiment, the bacterial culture comprises independent or a mixture of the strains ATTC 53582, and/or ATCC 11142 and/or DSM 14666.

In the method of synthesis of BNC, it is possible to control the resulting pore/mesh sizes. In order to achieve this control of the pore/mesh sizes additional substances are added to the fermentation medium. The modification allows specifically tailoring the BNC for the microorganism to be loaded. As a remarkable benefit of the bacterial cellulose, the property-controlling fiber network and the pore system formed by self-assembly of the cellulose molecules can be modified in situ using additives during biosynthesis. The additives are selected from polyethylene glycol (PEG), b-cyclodextrin, carboxymethyl cellulose (CMC), methyl cellulose (MC), cationic starches such 2-hydroxy-3-trimethylammoniumpropyl starch chloride, and TMAP starch. The addition of polyethylene glycol (PEG) 4000 causes a pore size decrease. In presence of b-cyclodextrin or PEG 400 remarkably increased pores can be achieved. The further advantage of these additives are, these co-substrates act as removable auxiliaries not incorporated in the BC samples.

**Pharmaceutically active ingredient (b1)**

Any pharmaceutically active ingredient or mixtures of pharmaceutically active ingredients known to the skilled person may be incorporated in the pharmaceutical compositions and S-SNEDDS. However, the pharmaceutical compositions of the present invention are very useful for poorly water-soluble pharmaceutically active ingredients, or for pharmaceutically active ingredients which show a high drug loss after storage. Preferably, the pharmaceutically active ingredient may be a poorly soluble, in particular poorly water-soluble, after peroral administration.

The pharmaceutically active ingredient (b1) may show a solubility of less than 0.1 mg of pharmaceutically active ingredient, preferably of the pure pharmaceutically active ingredient, in 1 ml water at 37 °C (as defined for poorly insoluble drugs in the USP). For instance, an excess amount of the pharmaceutically active ingredient is placed in a certain amount of water and mixed. The dissolved amount of the pharmaceutically active ingredient is then determined by a suitable analytical method, for instance by spectrometry.

In one embodiment the at least one pharmaceutically active ingredient may be selected from acalabrutinib, albendazole, allendronic acid, aripiprazole, asenapine, atazanavir, atorvastatin, BETd-260 bleomycin, bosentan, BRD4 degrader AT1, buprenorphine, budesonide, camostat, candesartan, carbamazepine, carvedilol, celecoxib, cilazapril, clarithromycin, clodronic acid, clopidogrel, curcumin, cytarabine, darunavir, dasatinib, deferasirox, dexamethasone, dexlansoprazole, diclofenac, diltiazem, docetaxel, doxorubicin, duloxetine, dutasteride, efavirenz, elbasvir, eprosartan, erlotinib, estradiol, etidronic acid, etravirine, everolimus, ezetimibe, felodipine, fenofibrate, fluconazole, fluorouracil, foretinib-based PROTAC 7, glimepiride, grazoprevir, griseovulvin, hydrochlorothiazide, hydrocortisone, hydroxychloroquine, ibuprofen, imatinib, irbesartan, irinotecan, itraconazole, ivacaftor, ivermectin, ledipasvir, lamotrigine, linezolid, lisinopril, lopinavir, losartan, lumefantrine, mefloquine, mesalazine, methotrexate, metoprolol, modafinil, moexipril, morphine, mycophenolate, naloxone, nifedipine, nilotinib, nilvadipine, nitrendipine, olanzapine, olmesartan, omeprazole, ondansetron, paclitaxel, pamidronic acid, paracetamol, pemetrexed, perindopril, phenytoin, pibrentasvir, pioglitazone, prednisone, progesterone, quetiapine, raloxifene, raltegravir, ramipril, rebamipide, remdesivir, rilpivirine, risedronic acid, risperidone, ritonavir, rivaroxaban, rivastigmine, rosuvastatin, selegiline, sevelamer, sibutramine, sildenafil, simvastatin, sirolimus, sitagliptin, sofosbuvir, sorafenib, spirapril, sunitinib, tacrolimus, tadalafil, tamoxifen, telaprevir, telmisartan, tenoxicam, terbutaline, ticagrelor, tiludronic acid, trandolapril, troglitazone, umifenovir, valsartan, velpatasvir, vemurafenib, verapamil, ziprasidone, zoledronic acid and ZXH-3-26, or, where applicable, from pharmaceutically acceptable salt forms thereof or mixtures thereof.

Preferably, the at least one pharmaceutically active ingredient may be selected from celecoxib, efavirenz, fenofibrate or mixtures thereof.

**Nutraceutical ingredient (b2)**

It is evident to a skilled person that there is a broad overlap between the terms pharmaceutical and nutraceutical active ingredients, excipients and compositions respectively a pharmaceutical or a nutraceutical dosage form. Many substances listed as nutraceuticals may also be used as pharmaceutical active ingredients. Depending on the specific application and local authority legislation and classification, the same substance may be listed as a pharmaceutical or a nutraceutical active ingredient respectively a pharmaceutical or a nutraceutical composition or even both.

Nutraceuticals are often defined as extracts of foods claimed to have medical effects on human health. Thus, nutraceutical active ingredients may display pharmaceutical activities as well: Examples for nutraceutical active ingredients may be resveratrol from grape products as an antioxidant, soluble dietary fiber products, such as psyllium seed husk for reducing hypercholesterolemia, broccoli (sulphane) as a cancer preservative, and soy or clover (isoflavonoids) to improve arterial health. Thus, it is clear that many substances listed as nutraceuticals may also be used as pharmaceutical active ingredients.

Typical nutraceuticals or nutraceutical active ingredients that may be used as fill-in for the described polymer-coated hard shell capsules may also include probiotics and prebiotics. Probiotics are living microorganisms believed to support human or animal health when consumed. Prebiotics are nutraceuticals or nutraceutical active ingredients that induce or promote the growth or activity of beneficial microorganisms in the human or animal intestine.

Examples for nutraceuticals are resveratrol from grape products, omega-3-fatty acids or pro-anthocyanines from blueberries as antioxidants, soluble dietary fiber products, such as psyllium seed husk for reducing hypercholesterolemia, broccoli (sulphane) as a cancer preservative, and soy or clover (isoflavonoids) to improve arterial health. Other nutraceuticals examples are flavonoids, antioxidants, alpha-linoleic acid from flax seed, beta-carotene from marigold petals or antocyanins from berries. Sometimes the expression neutraceuticals or nutriceuticals are used as synonyms for nutraceuticals.

**Lipid component (c)**

Any lipid component which can be used for pharmaceutical compositions is in general suitable.

The at least one lipid component (c) can be selected from medium chain triglycerides ($C_6$-$C_{12}$ fatty acids), long chain triglycerides ($C_{13}$-$C_{21}$ fatty acids), propylene glycol dicaprylate / dicaprate (Captex® 200), glyceryl tricaprylate / tricaprate (Captex® 300), glyceryl triricinoleate (Castor oil), medium chain triglycerides (lauric acid) (Coconut oil), glyceryl dibehenate (Compritol® 888 ATO), triglycerides (linoleic acid, oleic acid) (Corn oil), triglycerides (linoleic acid, oleic acid, palmitic acid) (Cottonseed oil), ethyl oleate (Crodamol™ EO), glyceryl tricaprylate / tricaprate (Crodamol™ GTCC), isopropyl myristate (IPM-100), glyceryl tricaprylate / tricaprate (Labrafac™ CC), glyceryl tricaprylate / tricaprate (Labrafac™ lipophil WL 1349), propylene glycol dicaprylate / dicaprate (Labrafac™ PG), long chain triglycerides / diglycerides / monoglycerides (monolinoleate) (Maisine® CC), glyceryl tricaprylate / tricaprate / trilaurate (Miglyol® 812), oleic acid (Pamolyn™ 100 Oleic Acid), triglycerides (oleic acid, palmitic acid) (olive oil), triglycerides (palmitic acid, oleic acid, linoleic acid), triglycerides (oleic acid, linoleic acid, palmitic acid), triglycerides (linoleic acid, oleic acid, palmitic acid) (palm oil),triglycerides (linoleic acid, oleic acid, alpha-linolenic acid, palmitic acid) (sesame oil), triglycerides (linoleic acid, oleic acid, stearic acid) (soybean oil), glyceryl triacetate (Kollisolv® GTA), glyceryl tricaprylate (Tricaprylin®), hard fat (triglycerides / diglycerides) and hard fat (triglycerides) (Witepsol® H 35) or any mixture thereof.

Expressions in the above list with brackets indicate the main components of the lipid component (example: medium chain triglycerides (lauric acid)). Expressions in the above list with slashes indicate the components of the lipid component (example: glyceryl tricaprylate / tricaprate / trilaurate).

**Surfactant (d)**

Any surfactant which can be used for pharmaceutical compositions is in general suitable.

The at least one surfactant (d), can comprise one or more surfactants and can be selected from polyoxyethylene (23) lauryl ether, polyoxyethylene (2) oleyl ether, glyceryl monooleate, medium chain monoglycerides / diglycerides (caprylate, caprate), glyceryl monocaprylate, propylene glycol monocaprylate, propylene glycol monocaprylate, polyoxyl-35 hydrogenated castor oil, polyoxyl-40 hydrogenated castor oil, lauroyl polyoxyl-32 glycerides, stearoyl polyoxyl-32 glycerides, polyoxyl-15 hydroxystearate, poloxamer 188 (triblock copolymer of polyoxyethylene and polyoxypropylene), poloxamer 407 (triblock copolymer of polyoxyethylene and polyoxypropylene), oleoyl polyoxyl-6 glycerides, linoleoyl polyoxyl-6 glycerides, lauroyl polyoxyl-6 glycerides, caprylocaproyl polyoxyl-8 glycerides, propylene glycol monolaurate (type II), propylene glycol monolaurate (type I), polyoxyl-40 stearate, diacetylated monoglyceride, glyceryl monooleate, polyglyceryl-3 dioleate, sorbitan monolaurate, sorbitan monooleate, sorbitan sesquioleate, sorbitan trioleate, glyceryl monostearate, d-alpha-tocopherol polyethylene glycol 1000 succinate (d-TPGS), polyoxyethylene sorbitan monolaurate, polyoxyethylene sorbitan monostearate and polyoxyethylene sorbitan monooleate or any mixture thereof.

**Solvent (e)**

A solvent can be employed in the present invention. Any solvent which can be used for pharmaceutical compositions is in general suitable.

The at least one solvent (e) can be selected from diethylene glycol monoethyl ether, polyethylene glycol 200, polyethylene glycol 400, polyethylene glycol 6000, propane-1,2,3-triol, (z)-octadec-9-enylamine, polypropylene glycol, propylene glycol, 2-pyrrolidone and tetraethylene glycol or any mixture thereof.

In an embodiment, the composition according to present invention is free of any solvent.

### Additive (f)

Any additive which can be used for pharmaceutical compositions, different from (a) to (e), is in general suitable. In one embodiment essentially no additive is present.

Additives are preferably selected from antiadherents, like magnesium stearate; fillers, like lactose, mannitol, starches, cellulose and their derivatives; binders, like polyacrylates, starches, guar, xanthan, alginate, carrageenan, pectin, tragacanth, polysaccharides and their derivatives; flavors, like mint, cherry, anise, vanilla, raspberry; colors, like natural colorants, azo and xanthene compounds; pigments, like titanium dioxides, iron oxides, magnesium oxide; disintegrants, like starches, croscarmellose, crosslinked polyvinylpyrrolidone, sodium hydrogen carbonate preferably in combination with citric acid (for effervescent tablets); glidants, like silica gel, fumed silica, talc, magnesium carbonate, flow regulators, like highly dispersed silicon dioxide; antioxidants like vitamin A, vitamin E, vitamin C, retinyl palmitate, selenium, butylated hydroxy anisole, butylated hydroxytoluene; sweeteners, like sucrose, sorbitol, saccharin sodium, cyclamate, aspartame; and antistatic, like alkyl sulfonates or quaternary ammonium compounds preferably combined with polystyrene; or mixtures thereof.

### Use

The solid self-nanoemulsifying drug delivery systems (S-SNEDDS), of the present invention are suitable for use (method of use) as a medicament or nutraceutical product, which preferably enhances the solubility of the included pharmaceutically active ingredient compared to the pharmaceutically active ingredient alone in the treatment of a disease of a human or an animal subject.

In another embodiment the loading factor [g L-SNEDDS/ g adsorbent] is in a ratio in the range of 1:1 to 100:1. Preferably the loading factor [g L-SNEDDS/ g adsorbent] is in a ratio in the range of 1:1 to 20:1.

### Items:

    1. A composition comprising:

        a. biosynthetic cellulose,
        b. at least one pharmaceutically active ingredient, or at least one nutraceutical ingredient,
        c. at least one lipid, and
        d. at least one surfactant,

    wherein the at least one pharmaceutically active ingredient, or the at least one nutraceutical ingredient is at least partially adsorbed on the biosynthetic cellulose.

    2. The composition according to item 1, wherein the biosynthetic cellulose is a network polymer, or a fiber polymer.

    3. The composition according to any one of the items 1 to 2, wherein the biosynthetic cellulose has density in the range of 0.5 to 1.3 $g/cm^3$.

    4. The composition according to any one of the items 1 to 3, wherein the biosynthetic cellulose has surface area > 1 $m^2/g$ as measured according to DIN ISO 9277, preferably in the range of 5 to 200 $m^2/g$ as measured according to DIN ISO 9277 and most preferably in the range of 15 to 100 $m^2/g$ as measured according to DIN ISO 9277.

    5. The composition according to any one of the items 1 to 5, wherein the biosynthetic cellulose has weight average molecular weight (Mw) in the range of 100,000 to 2000,000 g/mol as measured according to GPC.

    6. The composition according to item 6, wherein the biosynthetic cellulose has weight average molecular weight (Mn) in the range of 500,000 to 780,000 as measured according to GPC.

    7. The composition according to any one of the items 1 to 7, wherein the biosynthetic cellulose has degree of crystallinity in the range of 0,5 to 0.95 % as measured according to solid state NMR spectroscopy.

8. The composition according to any one of the items 2 to 8, wherein the fiber biosynthetic cellulose has diameter in the range of 1 nm to 5 nm.

9. The composition according to any one of the items 2 to 9, wherein the fiber biosynthetic cellulose has length in the range of 1 nm to 1000 nm.

10. The composition according to any one of the items 1 to 10, wherein the at least one pharmaceutically active ingredient, or the at least one nutraceutical active ingredient has a solubility of less than 0,1mg in 1 ml water at 37 °C.

11. The composition according to any one of the items 1 to 11, wherein the at least one pharmaceutically active ingredient is selected from celecoxib, efavirenz, fenofibrate, or a mixture of two or more thereof.

12. The composition according to any one of the items 1 to 12, wherein the at least one lipid is selected from $C_6$-$C_{24}$ fatty acids or a slat thereof, $C_1$-$C_{24}$ alkyl esters of $C_1$-$C_{24}$ fatty acids, monoglycerides of $C_6$-$C_{24}$ fatty acids, diglycerides of $C_6$-$C_{24}$ fatty acids, triglycerides of $C_6$-$C_{24}$ fatty acids, mono or di esters of $C_6$-$C_{24}$ fatty acids with propylene glycol, hard fat, or a mixture of two or more thereof.

13. The composition according to any one of the items 1 to 13, wherein the at least one surfactant selected from polyalkylene glycol, C1-C24 alkyl ether of polyalkylene glycol, C1-C24 alkyl ester of polyalkylene glyol, polyoxyl-35 hydrogenated castor oil, polyoxyl-40 hydrogenated castor oil, diacetylated monoglyceride, sorbitan monolaurate, sorbitan monooleate, sorbitan sesquioleate, sorbitan trioleate d-a-tocopherol, polyethylene glycol 1000 succinate, polyoxyethylene sorbitan monolaurate, polyoxyethylene sorbitan monostearate, polyoxyethylene sorbitan monooleate, or a mixture of two or more thereof.

14. The composition according to any one of the items 1 to 14 comprises at least one solvent (e).

15. The composition according to item 15, wherein the at least one solvent (e) is selected from diethylene glycol monoethyl ether, polyethylene glycol 200, polyethylene glycol 400, polyethylene glycol 6000, propane-1 ,2, 3-triol, (z)-octadec-9- enylamine, polypropylene glycol, propylene glycol, 2-pyrrolidone, tetraethylene glycol and diethylene glycol monoethyl ether, or a mixture of two or more thereof.

16. The composition according to any one of the items 1 to 16 comprises at least one additive (f).

17. The composition according to item 17, wherein the at least one additive (f) is selected from antiadherents, binders, flavors, pigments, disintegrants, glidants, flow regulators, antioxidants, sweeteners, antistatics, or a mixture of two or more thereof.

18. The composition according to any one of the items 1 to 18, wherein the biosynthetic cellulose is present in an amount in the range of 5 to 99 wt. %, based on total weight of the composition.

19. The composition according to any one of the items 1 to 19, wherein the at least one pharmaceutically active ingredient, or the at least one nutraceutical ingredient is present in an amount in the range of 1 to 95 wt. % based on total weight of the composition.

20. The composition according to any one of the items 1 to 20, wherein the at least one lipid is present in an amount in the range of 5 to 50 wt. %, based on total weight of the L-SNEDD composition.

21. The composition according to any one of the items 1 to 21, wherein the at least one surfactant is present in an amount in the range of 20 to 70 wt. %, based on total weight of the L-SNEDD composition.

22. The composition according to anyone of the items 1 to 22, wherein the biosynthetic cellulose is prepared by agitated cultivation process, or static cultivation process.

23. The composition according to anyone of the items 1 to 23, wherein the biosynthetic cellulose is obtained from a microorganism.

24. The composition according to item 23, wherein the microorganism is a bacterial microorganism.

25. The composition according to item 24, wherein the bacterial microorganism is selected form the strains Acetobacter xylinus, K. xylinum, Gluconacetobacter hansenii NQ5 (Acetobacter xylinus Yamada) ATTC 53582, the strains of K. xylinum ATCC 11142, DSM 14666 or a mixture thereof.

26. A process for preparing a composition according to any one of the items 1 to 24 comprising at least the step of:

a. providing at least one pharmaceutically active ingredient, or at least one nutraceutical ingredient,

b. providing at least one lipid,

c. providing at least one surfactant,

d. mixing the components of step a., step b., and step c. to obtain a homogenous mixture, and

e. providing biosynthetic cellulose to the homogenous mixture obtained in step d. to obtain the product.

27. The process according to item 26 comprises a step
b1. converting the lipid of step b. to a liquid state by providing heat prior to mixing step d.

28. The process according to items 26 to 27 comprises a step
c1. converting the surfactant of step c. to a liquid state by providing heat prior to mixing step d.

29. A drug delivery system comprising a composition according to any one of the items 1 to 25.

30. Use of a composition according to any one of the items 1 to 25 as drug delivery system.

31. A method of delivering a pharmaceutically active ingredient, or a nutraceutical ingredient comprising at least the step of:
a. providing a composition according to any one the items 1 to 25.

**Materials and Methods**

**Materials**

The model drug celecoxib was obtained from Aarti Drugs Ltd. (Mumbai, India).

Methyl cellulose, polyoxyethylene (80) sorbitan monooleate (Tween® 80), and d-α-tocopherol polyethylene glycol 1000 succinate (d-TPGS) were purchased from Sigma Aldrich Chemie GmbH (Steinheim, Germany).

Gelucire® 44/14 was obtained from Gattefossé S.A.S (Saint Priest, France).

Miglyol® 812 was obtained from Caesar & Loretz GmbH (Hilden, Germany).

Aeroperl® 300 Pharma, Zeopharm® 600 and Sipernat® 160 PQ are obtained from Evonik Resource Efficiency GmbH (Hanau, Germany).

Biosynthesized Cellulose (static or agitated cultivation) are obtained from JeNaCell GmbH (Jena, Germany).

Syloid® XDP 3050 was obtained from W. R. Grace & Co.-Conn (Worms, Germany).

Avicel® PH-101 was obtained from Dow Chemical Company (Schwalbach am Taunus, Germany). Wood derived Cellulose Nanocrystals, Nanocrystacell CNC, were obtained from Nanocrystacell (Podcerkev, Slovenia).

Wood derived Cellulose Nanocrystals, and CelluForce NCC®, were obtained from CelluForce Inc. (Windsor, Canada). All other chemicals and solvents were of analytical grade and purchased commercially.

**Biosynthesized cellulose (agitated cultivation)**

Biosynthesized bacterial Cellulose (BC) fibers were produced by cultivation of Gluconacetobacter hansenii NQ5 (Acetobacter xylinus Yamada) ATTC 53582 in a standard lab scale fermenter. For cultivation the established HSM medium (Hestrin & Schramm, 1954) was used, supplemented with 0.5% Hydroxyethylcellulose. The medium was inoculated with 10% shake flask preculture. The following process parameters were set: temperature 28°C, pH setpoint 5.1 controlled by base addition, pO2 25% controlled by stirring and aeration, run time ~40 h.

The broth was harvested from the fermenter and the microorganism was inactivated by adding 0.1N NaOH and incubation at room temperature for minimum 48 h. Afterwards bacterial cellulose was washed and neutralized by sequential addition of water, centrifugation in a lab scale centrifuge at ~12000 g for 5 to 30 minutes and removal of the supernatant until the pH of the cellulose is neutral again. Biosynthetic bacterial cellulose (1-2 g/kg aqueous dispersion) was dried by lyophilization.

**Biosynthesized bacterial cellulose (static cultivation)**

Biosynthesized Bacterial Cellulose (BC) was prepared by static cultivation using the *Komagataeibacter xylinus* strain DSM14666, deposited at the German Collection of Microorganism and Cell Cultures (DSMZ, Braunschweig, Germany). *K. xylinus* was cultivated in Hestrin-Schramm culture medium. The inoculation was carried out in 400 cm$^2$ trays using a preculture of *K. xylinus.* For each inoculation the ratio between preculture and HSM was 1:20. The inoculated trays were maintained at 28 °C for 7 days for BC synthesis. During the incubation time, BC was synthesized as a growing biofilm on the surface of the medium. It was harvested and afterwards treated with 0.1M of an aqueous sodium hydroxide solution. Afterwards, BC was purified with deionized water until a neutral pH of 7 was obtained. Eventually, BC was sterilized via irradiation.

**Methods**

***Preparation of L-SNEDDS***

The L-SNEDDS formulation containing the drug substance celecoxib was prepared by accurately weighing all excipients and the drug substance into a glass beaker. If solid or semisolid excipients were included, they were melted by applying an elevated temperature of 50 °C. The excipients were subsequently homogenized under moderate magnetic stirring at 300 rpm for at least 30 min and were used for further processing.

***Adsorption to a solid carrier (preparation of S-SNEDDS)***

The adsorption to a solid carrier was performed to convert L-SNEDDS into S-SNEDDS. For this purpose, 1.0 g of the liquid phase (L-SNEDDS) comprising a combination of lipids/oils, surfactants, co-solvents and the dissolved drug substance, was placed in a 25 ml glass beaker under moderate stirring using a propeller stirrer at 100 rpm. The adsorptive carrier material (biosynthetic cellulose) was added to the liquid phase in portions and different ratios depending on the adsorptive capacities of the carrier material. Based on the mixture ratio considering the amount of L-SNEDDS and the solid carrier material, the loading factor for each specific carrier was calculated. The adsorption process has been completed as soon as a dry, solid and flowable material was obtained. The blend was gently and continuously stirred at 100 rpm for another 2 min to guarantee homogenized S-SNEDDS.

***HPLC method for analyzing celecoxib***

The high-performance liquid chromatography (HPLC) system (Agilent 1260 Infinity) was used for the quantification of celecoxib consisted of a quaternary pump (G1311B), autosampler (G1329B), column oven (G1316A) and UV detector (G1314C), all obtained from Agilent Technologies (Frankfurt am Main, Germany). Separation was achieved using a Knauer Nucleosil 100-7 C18 (125 × 4.6 mm, 7 $\mu$m) column maintained at 40 °C. The mobile phase consisted of an acetonitrile: water: triethylamine mixture (300:300:0.9 v/v), adjusted to pH 3.00 with phosphoric acid. The flow rate was set to 1.8 ml/min. An injection volume of 5 $\mu$l was applied and celecoxib was detected at 254 nm. In the concentration range of 0.12 - 558 $\mu$g/ml, the analytical curve was linear ($R^2$ = 0.999999). The method was found to be accurate (100.4 - 101.5%) and precise (CV 2.59%) with a quantification limit of 0.05 $\mu$g/ml. Run time was defined to be 7 min. Selectivity was determined (formulation excipients) and no interference was observed in drug retention time. Moreover, the celecoxib peak area did not change in the presence of all excipients used in the study.

***Dissolution test of S-SNEDDS***

Dissolution experiments were performed according to USP 42-NF 37 (2019) and were conducted with 25 mg celecoxib or an equivalent amount of S-SNEDDS using USP apparatus (DT 800 LH) from ERWEKA GmbH (Langen, Germany). The paddle speed was set to 100 rpm and all experiments were performed in 500 ml of 0.1N hydrochloric acid at 37 ± 0.5 °C. All samples that were withdrawn by a fraction collector, equipped with cannula filters of 10 $\mu$m pore size, during the dissolution test were diluted 1:1 (v/v) with acetonitrile and analyzed via HPLC as described.

The composition of L-SNEDDS for the drug substance celecoxib is shown in Table 1. Miglyol® 812 was used as the oily carrier and Tween® 80, Gelucire® 44/14 and d-TPGS as a surfactant mixture. In a next step the adsorption to a solid carrier material was applied as an elementary technology mainly based on capillary forces. After adsorption to a solid carrier, depending on the carrier properties, a particulate system with different flowability qualities was obtained. The loading capacity of the adsorptive carriers (Table 2) was determined according to the corresponding method described in the section "*Adsorption to a solid carrier (preparation of S-SNEDDS)*" by final calculation using the following equation (1):

$$loading\ factor = m(\text{L-SNEDDS}) / m(\text{adsorptive carrier}) \qquad (1)$$

The total drug load of S-SNEDDS as shown in Table 2 was calculated using the following equation:

$$total\ drug\ load\ (S\text{-}SNEDDS)\ in\ [\%]$$

$$= m(\text{celecoxib}) / m(\text{L-SNEDDS}) + m(\text{adsorptive carrier}) \qquad (2)$$

The evaluation of the loading factors for the adsorptive carrier materials revealed that the highest loading capacities for L-SNEDDS were achieved with biosynthetic cellulose materials (static or agitated cultivation). Biosynthetic cellulose (agitated cultivation) turned out to be the best-performing material regarding the loading capacity for L-SNEDDS and demonstrated the highest total drug load of S-SNEDDS (Table 2).

*Table 1: Final composition of L-SNEDDS incorporating celecoxib.*

|  | Miglyol® 812 | Tween® 80 | Gelucire® 44/14 | d-TPGS | celecoxib |
|---|---|---|---|---|---|
| amount [wt. %] | 30.27 | 49.85 | 4.55 | 6.24 | 9.09 |

*Table 2: Loading capacity of adsorbents and drug load of S-SNEDDS.*

| adsorbent | chemical term of adsorbent | loading factor [g L-SNEDDS/ g adsorbent] | total drug load of S-SNEDDS [%] | drug release after 120 min [%] |
|---|---|---|---|---|
| Aeroperl® 300 Pharma | colloidal silicon dioxide | 1.40 | 5.30 | 34.51 |
| Zeopharm® 600 | calcium silicate | 2.37 | 6.39 | 56.15 |
| Sipernat® 160 PQ | amorphous silicon dioxide | 2.44 | 6.45 | 62.39 |
| Syloid® XDP 3050 | amorphous silicon dioxide | 1.44 | 5.36 | 23.13 |
| Avicel® PH-101 | microcrystalline cellulose | 0.90 | 4.31 | 90.12 |
| Biosynthetic cellulose (agitated cultivation) | biosynthetic cellulose | 7.13 | 7.95 | 99.07 |
| Biosynthetic cellulose (static cultivation) | biosynthetic cellulose | 3.61 | 7.12 | 99.27 |
| Nanocrystacell CNC | nanocellulose (wood derived) | 0.35 | 2.37 | 99.73 |
| CelluForce NCC® | nanocellulose (wood derived) | 0.28 | 2.01 | 99.62 |

The celecoxib dose applied in the dissolution experiments was 25 mg or an equivalent amount S-SNEDDS. All S-SNEDDS based on biosynthetic cellulose materials displayed a drug release > 97% after 120 min of test duration (Table 2 & Figure 1) compared to the silica-based S-SNEDDS. The silica-based S-SNEDDS retained a proportionally larger part of its adsorbed drug substance celecoxib compared to the bacterial cellulose-based S-SNEDDS resulting in lower drug releases.

**Claims**

**1.** A composition comprising:

a. biosynthetic cellulose,
b. at least one pharmaceutically active ingredient, or at least one nutraceutical ingredient,
c. at least one lipid, and
d. at least one surfactant,

wherein the at least one pharmaceutically active ingredient, or the at least one nutraceutical ingredient is at least partially adsorbed on the biosynthetic cellulose.

2. The composition according to claim 1, wherein the biosynthetic cellulose has density in the range of 0.5 to 1.3 g/cm$^3$.

3. The composition according to any one of the claims 1 to 2, wherein the biosynthetic cellulose has weight average molecular weight (Mw) in the range of 100,000 to 2,000,000 g/mol as measured according to GPC.

4. The composition according to any one of the claims 1 to 3, wherein the at least one pharmaceutically active ingredient, or the at least one nutraceutical active ingredient has a solubility of less than 0.1mg in 1 ml water at 37 °C.

5. The composition according to any one of the claims 1 to 4, wherein the at least one pharmaceutically active ingredient is selected from celecoxib, efavirenz, fenofibrate, or a mixture of two or more thereof.

6. The composition according to any one of the claims 1 to 5, wherein the at least one lipid is selected from $C_6$-$C_{24}$ fatty acids or a slat thereof, $C_1$-$C_{24}$ alkyl esters of $C_1$-$C_{24}$ fatty acids, monoglycerides of $C_6$-$C_{24}$ fatty acids, diglycerides of $C_6$-$C_{24}$ fatty acids, triglycerides of $C_6$-$C_{24}$ fatty acids, mono or di esters of $C_6$-$C_{24}$ fatty acids with propylene glycol, hard fat, or a mixture of two or more thereof.

7. The composition according to any one of the claims 1 to 6, wherein the at least one surfactant selected from polyalkylene glycol, C1-C24 alkyl ether of polyalkylene glycol, C1-C24 alkyl ester of polyalkylene glyol, polyoxyl-35 hydrogenated castor oil, polyoxyl-40 hydrogenated castor oil, diacetylated monoglyceride, sorbitan monolaurate, sorbitan monooleate, sorbitan sesquioleate, sorbitan trioleate d-a-tocopherol, polyethylene glycol 1000 succinate, polyoxyethylene sorbitan monolaurate, polyoxyethylene sorbitan monostearate, polyoxyethylene sorbitan monooleate, or a mixture of two or more thereof.

8. The composition according to any one of the claims 1 to 7 further comprises at least one additive.

9. The composition according to any one of the claims 1 to 8, wherein the biosynthetic cellulose is present in an amount in the range of 5 to 99 wt. %, based on total weight of the composition.

10. The composition according to any one of the claims 1 to 8, wherein the at least one pharmaceutically active ingredient, or the at least one nutraceutical ingredient is present in an amount in the range of 1 to 95 wt. %, based on total weight of the composition.

11. The composition according to any one of the claims 1 to 10, wherein the biosynthetic cellulose is obtained from a bacterial microorganism.

12. The composition according to claim 11, wherein the bacterial microorganism is selected form are selected from the strains of Acetobacter xylinus, K. xylinum, Gluconacetobacter hansenii NQ5 (Acetobacter xylinus Yamada) ATTC 53582, the strains of K. xylinum ATCC 11142, DSM 14666 or a mixture thereof.

13. A process for preparing a composition according to any one of the claims 1 to 12 comprising at least the step of:

a. providing at least one pharmaceutically active ingredient, or at least one nutraceutical ingredient,
b. providing at least one lipid,
c. providing at least one surfactant,
d. mixing the components of step a., step b., and step c. to obtain a homogenous mixture, and
e. providing biosynthetic cellulose to the homogenous mixture obtained in step d. to obtain the product.

14. A drug delivery system comprising a composition according to any one of the claims 1 to 12.

15. A method of delivering a pharmaceutically active ingredient, or a nutraceutical ingredient comprising at least the step of:

a. providing a composition according to any one the claims 1 to 12.

Europäisches
Patentamt

European
Patent Office

Office européen
des brevets

**EUROPEAN SEARCH REPORT**

Application Number

EP 23 20 0805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SILVA L.A.D. ET AL.: "Preparation of a solid self-microemulsifying drug delivery system by hot-melt extrusion", INTERNATIONAL JOURNAL OF PHARMACEUTICS, vol. 541, no. 1-2, 1 April 2018 (2018-04-01), pages 1-10, XP093122020, NL ISSN: 0378-5173, DOI: 10.1016/j.ijpharm.2018.02.020 * the whole document * | 1-15 | INV. A61K9/107 A61K47/38 B01D39/18 C08L1/02 C12N15/09 C12P19/04 |
| A | WO 2017/203281 A1 (CUSTOMEM LTD [GB]) 30 November 2017 (2017-11-30) * examples * | 1-15 | |
| A | WO 2020/058836 A1 (INDIAN INSTITUTE OF TECH HYDERABAD [IN]) 26 March 2020 (2020-03-26) * example 6 * | 1-15 | |
| A | CN 110 251 488 B (UNIV HAINAN NORMAL) 20 August 2021 (2021-08-20) * examples * | 1-15 | TECHNICAL FIELDS SEARCHED (IPC)  A61K C12P C09J C40B B01D C08L C12N |

The present search report has been drawn up for all claims

| Place of search | Date of completion of the search | Examiner |
|---|---|---|
| Munich | 22 January 2024 | Giménez Miralles, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

EP 4 529 919 A1

## ANNEX TO THE EUROPEAN SEARCH REPORT
## ON EUROPEAN PATENT APPLICATION NO.

EP 23 20 0805

This annex lists the patent family members relating to the patent documents cited in the above-mentioned European search report.
The members are as contained in the European Patent Office EDP file on
The European Patent Office is in no way liable for these particulars which are merely given for the purpose of information.

22-01-2024

| Patent document cited in search report | | Publication date | Patent family member(s) | | Publication date |
|---|---|---|---|---|---|
| WO 2017203281 | A1 | 30-11-2017 | GB | 2551044 A | 06-12-2017 |
| | | | WO | 2017203281 A1 | 30-11-2017 |
| WO 2020058836 | A1 | 26-03-2020 | US | 2022040115 A1 | 10-02-2022 |
| | | | WO | 2020058836 A1 | 26-03-2020 |
| CN 110251488 | B | 20-08-2021 | NONE | | |

EPO FORM P0459

For more details about this annex : see Official Journal of the European Patent Office, No. 12/82

14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2101729 B1
- CN 107308133 A

- EP 2547372 A1

**Non-patent literature cited in the description**

- **SILVA,** ; **D. LUIS ANTONIO et al.** *International Journal of Pharmaceutics*, 2018, vol. 541, 1-10